(19) Europäisches Patentamt
European Patent Office
Office européen des brevets

(11) **EP 2 129 765 B1**

(12) **EUROPÄISCHE PATENTSCHRIFT**

(45) Veröffentlichungstag und Bekanntmachung des Hinweises auf die Patenterteilung:
**07.05.2014 Patentblatt 2014/19**

(21) Anmeldenummer: **08715932.3**

(22) Anmeldetag: **21.02.2008**

(51) Int Cl.:
***C12M 1/107*** (2006.01)    ***C12M 1/06*** (2006.01)

(86) Internationale Anmeldenummer:
**PCT/EP2008/001372**

(87) Internationale Veröffentlichungsnummer:
**WO 2008/104320 (04.09.2008 Gazette 2008/36)**

(54) **BIOGASANLAGEN-FERMENTER MIT EINEM MOTORISCH HÖHENVERSTELLBAREN TAUCHMOTORRÜHRGERÄT**

FERMENTER FOR BIOGAS PLANTS, COMPRISING A SUBMERSIBLE MOTOR-DRIVEN STIRRER VERTICALLY ADJUSTABLE BY MEANS OF A MOTOR

FERMENTEUR POUR INSTALLATIONS À BIOGAZ, COMPRENANT UN AGITATEUR À MOTEUR SUBMERSIBLE RÉGLABLE EN HAUTEUR DE MANIÈRE MOTORISÉE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **27.02.2007 DE 102007009451**

(43) Veröffentlichungstag der Anmeldung:
**09.12.2009 Patentblatt 2009/50**

(73) Patentinhaber: **UTS BIOGASTECHNIK GmbH 84419 Schwindegg (DE)**

(72) Erfinder: **BÜRGER, Adam 83527 Haag (DE)**

(74) Vertreter: **Schütte, Hartmut et al BSB Anwaltskanzlei Am Markt 2 (Eingang Herrenstrasse) 59302 Oelde (DE)**

(56) Entgegenhaltungen:
**EP-A- 1 130 084       EP-A- 1 256 373
DE-A1- 4 120 987      DE-C1- 4 015 478
DE-C1- 19 517 901     DE-U-202004 004 101
DE-U1- 8 605 277      GB-A- 2 110 550**

• **DATABASE WPI Week 198837, Derwent Publications Ltd., London, GB; AN 1988-260388 & JP S63 188 384 A (RIKAGAKU KENKYUSHO) 03 August 1988**

**Beschreibung**

Die Erfindung betrifft einen Biogasanlagen-Fermenter mit einem motorisch höhenverstellbaren Tauchmotorrührgerät nach dem Oberbegriff des Anspruchs 1.

In Biogasanlagen läuft ein Fermentationsprozess ab, bei dem organische Stoffe, wie beispielsweise Wirtschaftsdünger aus der Landwirtschaft (Rindergülle, Rinder-Festmist, Schweinegülle, Schweine-Festmist, Hühnergülle, Hühner-Trockenkot) und/oder landwirtschaftliche Reststoffe (Grasschnitt-Rübenblätter, Silagen) und/oder Reststoffe aus der Agroindustrie oder verwandten Industrien (Biertreber, Obstreste, Gemüsereste, Rapsschrot, Getreideabputz, Schlempen, Melasse) als Biomasse vergast werden. Die hierbei entstehenden Gase sammeln sich in einem oberen Fermenterbehälterbereich eines Fermenterbehälters und können direkt zur Energieerzeugung verwendet werden, z. B. als Heizgas zur Stromerzeugung in nachgeschalteten Brennkraftmaschinen mit Elektrogeneratoren. Zur Fermentation werden im Fermenterbehälter die organischen Stoffe mit Flüssigkeit versetzt und der Fermentations- bzw. der Vergasungsprozess unter aeroben oder anaeroben Bedingungen durch Mikroorganismen, wie z.B. Hefen, Bakterien, etc. durchgeführt.

DE 86 05 277 U offenbart in einem Reaktor einen Tauchmotor-Mixer, der eine freie Auf- und Abwärtsbewegung im Flüssigkeitsbehälter ausführen kann. Der Antriebsmotor ist variabel einstellbar, je nach Viskosität, ebenso die Auf- und Abwärtsbewegungen des Rührwerks.

US5867972 offenbart einen Weiher, dessen Flüssigkeitsdurchfluss mittels Füllhöhensensor gemessen wird und dementsprechend auf der Basis des Sensorsignals (konduktives Signal) die Weiherkante (weir lip) abgesenkt oder angehoben werden kann. JP63188384 offenbart einen Bioreaktor zur kontinuierlichen Kultivierung von Zellen in hoher Dichte. Die Vorrichtung beinhaltet einen Rührer, der fest installiert ist und einen Füllstandssensor mit dessen Hilfe frisches Medium zudosiert und über ein Ventil Kulturlösung nach Maßgabe voreingestellter Sollwerte abgeführt werden kann.

Ein Problem bei derartigen allgemein bekannten Fermentationsprozessen ist, dass die Biomasse, insbesondere Biomasse-Feststoffe in der Fermenterflüssigkeit regelmäßig nicht gleichmäßig verteilt sind, da sie z. B. aufschwimmen und sich im Bereich der Flüssigkeitsoberfläche ansammeln. Andererseits kann aber auch das Problem bestehen, dass Biomasse, insbesondere Biomasse-Feststoffe mit einem höheren spezifischen Gewicht als die Fermenterflüssigkeit auf den Fermenterbehälterboden absinken und sich dort ungünstig als Sinkschichten ansammeln. Allgemein ist für einen hohen Wirkungsgrad des Fermentationsprozesses eine möglichst gleichmäßige Biomasseverteilung in der Fermenterflüssigkeit erforderlich.

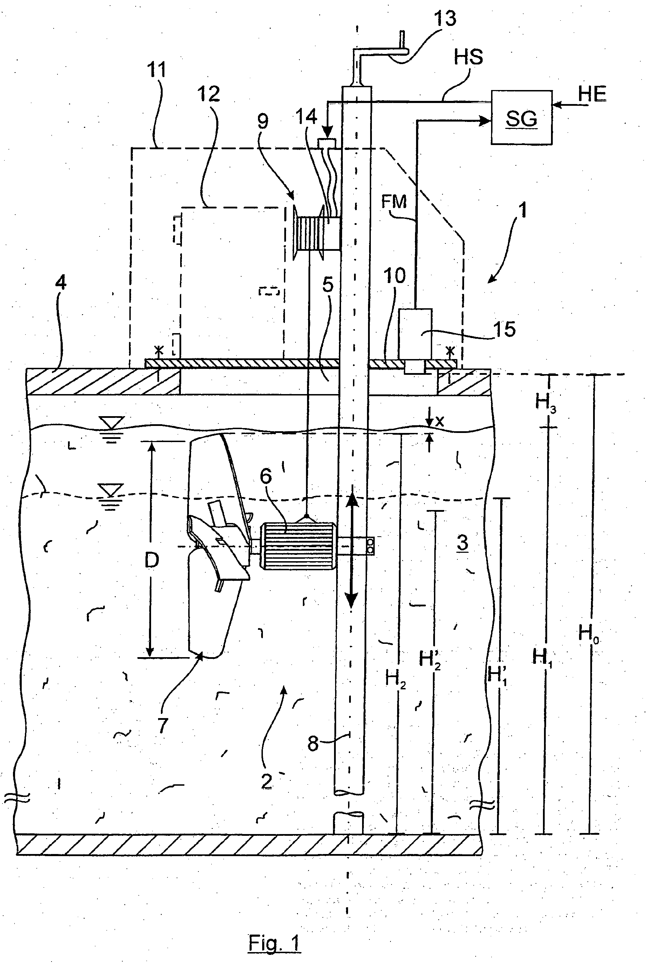

Für eine solche möglichst gleichmäßige Verteilung der Biomasse in der Fermenterflüssigkeit zur Steigerung des Wirkungsgrades ist bereits eine gattungsbildende Rühreinrichtungen bekannt (DE 197 32 198 C1, Fig. 1). Die Rühreinrichtung umfasst hier ein Tauchmotorrührgerät, das an einem vertikal im Behälter angeordneten Aggregatträger mittels einer durch einen Elektromotor gesteuerten Seilwinde höhenverstellbar gehalten ist. Das Tauchmotorrührgerät enthält einen Tauchmotor mit einer etwa horizontal liegenden Rührerwelle, an der Rührflügel angebracht sind. Weiter ist es aus dieser Druckschrift bekannt, das Tauchmotorrührgerät zu Wartungs- und/oder Reparaturzwecken mittels der Seilwinde in einen als Dom ausgeführten Serviceschacht durch eine Wartungsöffnung in der Fermenterdeckenwand nach oben heraus zu ziehen. Dies ist vorteilhaft möglich, ohne dass der Flüssigkeitsspiegel im Fermenter abgesenkt werden muss und damit der Fermentationsprozess gestört wird.

Im Anlagenbetrieb wird das Tauchmotorrührgerät je nach den Rührerfordernissen durch Ansteuerung des zugeordneten Höhenstellmotors von Hand oder gegebenenfalls nach einem bestimmten Zeitablaufplan höhenverstellt. Je nach Ablauf des Fermentationsvorgangs in Verbindung mit einer meist automatisieren Zudosierung von Biomasse, kann die Füllhöhe des Fermenters schwanken. Dadurch kann bei einer Höheneinstellung des Tauchmotorrührgeräts im oberen Fermenterbereich ein Zustand auftreten, bei dem die Rührflügel aus dem Substrat während des Rührvorgangs zumindest teilweise austauchen. Dadurch werden nachteilig die Durchmischung des Substrats und damit das Rührergebnis reduziert, wodurch insgesamt auch die Biogasausbeute reduziert wird. Zudem verspritzen austauchende Rührflügel das Substrat über das Substratniveau hinaus, was zu Verunreinigung beispielsweise von Sichtfenstern führen kann. Bei insgesamt eingetauchten Rührflügeln ist die Energieaufnahme und mechanische Belastung des Tauchmotorrührgeräts etwa gleichmäßig. In der Austauchphase eines Rührflügels oder Rührflügelteils ist dagegen die mechanische Belastung dieses Rührflügels reduziert und beim Eintauchen wieder erhöht, so dass der Antriebsmotor, die Lager und Wellen entsprechend periodisch ungleichmäßig belastet werden. Dies erhöht insbesondere bei einem Substrat mit hoher Biomasse-Feststoffkonzentration den Verschleiß an einem Tauchrührgerät und kann dessen Standzeit erheblich reduzieren.

Aufgabe der Erfindung ist es daher, einen gattungsgemäßen Biogasanlagen-Fermenter mit einem motorisch höhenverstellbaren Tauchmotorrührgerät so weiterzubilden, dass auch bei betriebsmäßig schwankendem Substrätniveau ein gleichmäßig gutes Rührergebnis bei weitgehend gleichmäßiger Belastung eines Tauchmotorrührgeräts vorliegt.

Diese Aufgabe wird mit den Merkmalen des Anspruchs 1 gelöst.

Gemäß Anspruch 1 ist am Fermenter eine Füllstands-Messvorrichtung vorgesehen, mit der die Füllhöhe des Substrats erfasst und ein entsprechendes Füllstands-Messsignal als Höhen-Istwertsignal erzeugt . wird. Zudem ist eine Steuereinrichtung vorgesehen, der das Füllstands-Messsignal zugeführt wird und die bei einer erfassten geringeren Füllhöhe, welche bezüglich der Voreinstellung zu einem Austauchen der Rührflügel führen würde, den Höhenstellmotor für das Tauchmotorrührgerät zu ansteuert, dass dieses soweit abgesenkt wird, so dass die Rührflügel weiter vollständig im Substrat eingetaucht sind.

Erfindungsgemäß wird somit selbsttätig und automatisiert auch bei einer relativ hohen Voreinstellung des Tauchmotorrührgeräts sichergestellt, dass auch bei einem betriebsbedingten Absinken des Substratniveaus die Rührflügel ständig eingetaucht bleiben. Dadurch treten die eingangs erläuterten Nachteile bei austretenden Rührflügeln nicht auf und ein gleichmäßiges Rührergebnis bei weitgehend gleichmäßiger Tauchmotorrührgerätbelastung ist gewährleistet.

Als Füllstands-Messvorrichtungen können je nach dem Fermenteraufbau und den weiteren Gegebenheiten unterschiedliche Systeme benutzt werden. Gemäß Anspruch 2 sind mechanisch arbeitende Sensoren mit Schwimmern oder Verdrängungskörpern in Verbindung mit Stellungsgebern und Grenzwertschaltern zur Erzeugung elektrischer Messsignale möglich. Besondern geeignet sind jedoch auch nach Anspruch 3 berührungslos arbeitende füllstands-Messvorrichtungen mit optischen und/oder akustischen und/oder radioaktiven Sensoren. Insbesondere können Echolot-Ultraschallimpulsgeräte oberhalb des Substratniveaus, beispielsweise in der Fermenterabdeckung oder auch an sich bekannte Gammastrahlern mit Zählrohrketten eingesetzt werden, an denen jeweils ein Messumformer zur Umformung des erhaltenen Messsignals in ein entsprechendes elektrisches Signal nachgeschaltet sind. Gegebenenfalls sind auch an sich bekannte hydrostatisch nach der Einperlmethode arbeitende Füllstands-Messvorrichtungen einsetzbar.

Mit den Merkmalen des Anspruchs 4 wird eine geeignete konkrete Anordnung beansprucht mit einem Sensor der Füllstands-Messvorrichtung oberhalb des Substratniveaus, vorzugsweise an einer Fermenterabdeckung, womit die Freiraumhöhe ($H_3$) zwischen dem berührungslos arbeitenden Sensor und dem Substratniveau durch ein Freiraumsignal (FM) erfasst wird. Dieses Freiraumsignal (FM) wird einem Steuergerät (SG) der Steuereinrichtung zugeführt, wo durch Substraktion von der Gesamthöhe ($H_0$) des Fermenterinnenraums die aktuelle Füllstandshöhe $H_1$ durch $H_1 = H_0 - H_3$ ermittelt wird. Gegebenenfalls können die aktuelle Füllstandshöhe ($H_1$) und ein entsprechendes Signal direkt, z. B. mit einer Schwimmeranordnung ermittelt werden. Über einen Höheneinsteller am Steuergerät bzw. einen entsprechenden Signaleingang wird ein Höheneinstellsignal (HE) entsprechend einem Sollwert ($H_2$) für das Tauchgerät dem Steuergerät (SG) zugeführt. Dieses steuert mit einem Höhenstellsignal (HS) den Höhenstellmotor so an, dass das Tauchmotorrührgerät diese Sollwerthöhe ($H_2$) (gemessen bis zur obersten Rührflügelspitze) einnimmt.

Der vorstehenden Höhensteuerung ist die nun folgende Sicherheitssteuerung gegen ein Austauchen der Rührflügel überlagert: Dazu wird im Steuergerät (SG) ein Vergleich zwischen den Signalen der aktuellen Füllstandshöhe ($H_1$) und der Sollwerthöhe ($H_2$) durchgeführt, dergestalt dass für den Fall einer Füllstandshöhe ($H_1$) größer als die Sollwerthöhe ($H_2$) kein weiterer Steuereingriff erfolgt, jedoch für den Fall, dass eine aktuell erfasste Füllstandshöhe ($H_1$') kleiner als die Sollwerthöhe ($H_2$) ist, das Tauchrührgerät nach unten abgesenkt wird. Die Absenkung erfolgt soweit, bis die aktuelle Einstellhöhe ($H_2$') des Tauchmotorrührgeräts um einen Höhendifferenzbetrags X kleiner als die aktuelle Füllstandshöhe ($H_1$') ist. Durch diese Bedingung ist sichergestellt, dass die Rührflügel auch bei dieser betriebsmäßig abgesenkten Substratfüllstandshöhe vollständig eingetaucht bleiben. Der Höhendifferenzbetrag X soll dabei so gewählt werden, dass die Rührflügelspitzen so weit unter dem Substratniveau drehen, das das Substrat nicht aufspritzt und ein gleichmäßiger Rührvorgang gewährleistet ist. Dazu wird mit Anspruch 5 vorgeschlagen, dass dieser Höhendifferenzbetrag X am Steuergerät (SG) vorgebbar ist und gemäß Anspruch 6 der überlagerte Steuereingriff für die Eintauchsicherung der Rührflügel jeweils beim Überschreiten einer X-Wertdifferenz durchgeführt wird. Dadurch kann sichergestellt werden, dass der Steuereingriff für die Eintauch-sicherung vorteilhaft nicht ständig in einem kritischen Bereich arbeitet, sondern in größeren Schritten arbeitet.

Das Steuergerät (SG) ist vorstehend als separates Gerät beschrieben worden, kann jedoch gemäß Anspruch 7 mit seiner Steuerfunktion für die Eintauchsicherung der Rührflügel in die Anlagengesamtsteuerung oder in einen Anlagensteuerungsteil integriert sein. Ebenso kann eine Füllstands-Messvorrichtung auch für andere Zwecke, beispielsweise für eine Feststoffzudosierung, mitverwendet werden.

In einer konkreten Ausführungsform nach Anspruch 8 ist das Tauchmotorrührgerät an einem vertikalen Aggregateträger geführt und mittels einer-Seilwinde motorisch höhenverstellbar gehalten. Die motorische Höhenverstellung kann auch mit anderen an sich bekannten Maßnahmen durchgeführt, beispielsweise auch an Seitenrührwerken, bei denen ein Tauchmotorrührwerk an einer höhenverschwenkbaren Schwinge angeordnet ist. Der Dreh-Antriebsmotor für ein Tauchmotorrührwerk kann ein Elektro-, Hydraulik- oder Pneumatikmotor sein.

In einer bewährten Anordnung nach Anspruch 9 ist der Aggregateträger im oberen Bereich in einem domartigen Serviceschacht aufgenommen, in den das Tauchrührgerät, insbesondere für Wartungs- und/oder Reparaturarbeiten nach oben gezogen werden kann und dort einfach zugänglich ist.

Anhand einer Zeichnung wird die Erfindung weiter erläutert.

Die einzige Figur zeigt schematisch eine Schnittdarstellung durch einen Biogasanlagen-Fermenter 1 mit einer Rühreinrichtung 2. Im Fermenter 1 ist ein vergärbares Substrat 3 mit einem Flüssigkeits- und Biomassefeststoffanteil enthalten. Der Fermenter 1 weist hier einen Betondecke 4 als Fermenterdeckenwand auf, könnte jedoch auch mit einem an sich bekannten Foliendach abgedeckt sein. In der Betondecke ist eine Wartungs- und Montageöffnung 5 angebracht. Die Rühreinrichtung 2 besteht aus einem Tauchmotorrührgerät 6 mit einer horizontal ausgerichteten Rührerwelle, an der hier in einer dreiflügeligen Anordnung Rührflügel 7 angebracht sind.

Das Tauchmotorrührgerät 6 ist an einem vertikalen Aggregateträger höhenverstellbar über eine Seilwinde 9 gehalten.

Über der durch eine Abdeckplatte 10 abgedeckten Wartungs- und Montageöffnung 5 ist ein (nur schematisch strichliert eingezeichnet) gasdicht angebrachter Serviceschacht 11 mit einer Tür 12 angeordnet. Der Aggregateträger 8 geht durch diesen Serviceschacht 11 hindurch und kann mit einer Kurbel 13 für eine Winkelverstellung des Tauchmotorrührgeräts 6 verdreht werden. Zudem ist die Seilwinde 9 im oberen Bereich des Serviceschachts 11 am Aggregateträger 8 angebracht, so dass nach Entfernen der Abdeckplatte 10 das Tauchmotorrührgerät 6 zu Wartungs- und Reparaturzwecken in den Bereich des Serviceschachts 11 nach oben gezogen werden kann. Die Höhenverstellung des Tauchmotorrührgeräts 6 wird durch eine entsprechende Ansteuerung eines mit der Seilwinde 9 gekoppelten elektrisch betriebenen Höhenstellmotors 14 durchgeführt. Die Ansteuerung erfolgt von einem Steuergerät SG, welches ein entsprechendes Höhen-Stellsignal HS an den Höhenstellmotor 14, beispielsweise an einen elektrischen Schrittmotor, abgibt. Die gewünschte Höheneinstellung des Tauchmotorrührgeräts 6 wird am Steuergerät SG durch ein Höhen-Einstellsignal HE vorgegeben. Dieses Höhen-Einstellsignal HE kann beispielsweise von Hand durch einen Handsteller oder auch im Rahmen eines Rührprogramms mit wechselnden Höheneinstellungen vorgegeben werden.

Im aktuell dargestellten Fall ist die Füllhöhe des Fermenters 1 bzw. das Substratniveau mit $H_1$ eingezeichnet. Die aktuelle Höheneinstellung des Tauchmotorrührgeräts 6 (bis zur obersten Spitze eines Rührflügels 7 entsprechen dem Durchmesser D der Rührflügelanordnung) ist entsprechend der Vorgabe des Höheneinstellsignals HE mit $H_2$ eingezeichnet. Die Rührflügel 7 sind dabei ersichtlich insgesamt während einer Rührfunktion im Substrat 3 eingetaucht, wobei ein Höhendifferenzbetrag X zwischen der obersten Rührflügelstellung und dem Substratspiegel eingehalten ist.

Der Fermentationsprozess hängt von einer Vielzahl von Parametern, wie beispielsweise der Art der Biomasse, der Zudosierung, der Temperatur, der Rührintensität usw. ab, so dass der Substratfüllstand betriebsmäßig merklich schwanken kann. Eine solche betriebsmäßige Schwankung mit einem abgesenkten Substratniveau auf die Füllhöhe $H_1$' ist in der Figur strichliert eingezeichnet. Ersichtlich würden nun die Rührflügel 7 des Tauchmotorrührgeräts 6 bei der Höheneinstellung $H_2$ während eines Rührvorgangs nachteilig über das Substratniveau hinaus austauchen. Um dies zu verhindern, ist zusätzlich eine Füllstandsmessvorrichtung 15, hier in der Art eines Ultraschall-Echolots im Bereich der Fermenterabdeckung, hier beispielsweise an der Abdeckplatte 10 angeordnet, wodurch die Freiraumhöhe $H_3$ zwischen der Füllstandsmessvorrichtung 15 und dem Substratniveau erfasst und ein entsprechendes elektrisches Freiraumhöhensignal FM dem Steuergerät SG zugeführt wird. Dort wird durch Substraktion der veränderlichen Freiraumhöhe $H_3$ von der gleichbleibenden Gesamthöhe $H_0$ die veränderliche aktuelle Füllstandshöhe $H_1$ ermittelt und erfasst.

Für den Fall, dass die Füllstandshöhe $H_1$ größer als die eingestellte Sollwerthöhe $H_2$ entsprechend dem Höheneinstellsignal HE ist, sind die Rührflügel 7 für ein gutes Rührergebnis vollständig eingetaucht. Dagegen erfolgt ein überlagerter Steuerungseingriff, wenn entsprechend der Figur das Substratniveau auf eine (strichliert eingezeichnete) Höhe $H_1$' absinkt, so dass dann die Rührflügel 7 austauchen würden. Dafür wird im Steuergerät ein Vergleich zwischen der aktuellen Höheneinstellung des Tauchmotorrührgeräts 6, die im Steuergerät SG erfasst ist, und der aktuell gemessenen, abgesenkten Füll-standshöhe $H_1$' vorgenommen. Wenn die abgesenkte Füllstandshöhe $H_1$' kleiner als die aktuelle Höhenlage des Tauchmotorrührgeräts 6 ist, gibt das Steuergerät SG ein Höhenstellsignal HS für einen Absenkvorgang des Tauchmotorrührgeräts 6 ab, solange bis die aktuelle Einstellhöhe $H_2$' wieder um einen Höhendifferenzbetrag X kleiner als die aktuelle Füllstandshöhe $H_1$' ist. Dadurch wird selbsttätig und automatisiert sichergestellt, dass während eines Rührvorgangs die Rührflügel 7 nicht über das Substratniveau hinaus austauchen.

**Patentansprüche**

1. Biogasanlagen-Fermenter mit einem motorisch höhenverstellbaren Tauchmotorrührgerät, wobei im Betrieb der Biogasanlage der Fermenter (1) mit einer Füllhöhe ($H_1$) mit zu vergärendem Substrat (3) befüllt ist und das Tauchmotorrührgerät (6) mit seinen Rührflügeln (7) vollständig im Substrat (3) eingetaucht ist,

   **dadurch gekennzeichnet,**

   **dass** eine Füllstands-Messvorrichtung (15) vorgesehen ist, mit der die Füllhöhe ($H_1$) des Substrats (3) erfasst und ein entsprechendes Füllstands-Messsignal (FM) als Höhen-Istwertsignal erzeugt wird,

   **dass** eine Steuereinrichtung (SG) vorgesehen ist, der das Füllstands-Messsignal (FM) zugeführt wird und die bei einer erfassten geringeren Füllhöhe ($H_1$') den Höhenstellmotor (14) für das Tauchmotorrührgerät (6) so ansteuert, dass dieses abgesenkt wird und die Rührflügel (7) weiter vollständig im Substrat eingetaucht sind.

2. Biogasanlagen-Fermenter nach Anspruch 1, **dadurch gekennzeichnet, dass** die Füllstands-Messvorrichtung (15) einen mechanisch arbeitenden Sensor mit einem Schwimmer und/oder einem Verdrängerkörper jeweils mit einem Stellungsgeber und/oder einem Grenzwertschalter umfasst.

3. Biogasanlagen-Fermenter nach Anspruch 1 oder Anspruch 2, **dadurch gekennzeichnet, dass** die Füllstands-Messvorrichtung (15) einen optischen und/oder akustischen und/oder radioaktiven Sensor, insbesondere ein Echolot-Ultraschallimpulsgerät oder einen berührungslos arbeitenden Gammastrahler mit Zählrohrkette und/oder ein Hydrostatikrohr für eine Einperlmethode umfasst.

4. Biogasanlagen-Fermenter nach einem der Ansprüche 1 bis 3, **dadurch gekennzeichnet, dass** ein Sensor der Füllstands-Messvorrichtung (15) oberhalb des Substratniveaus, vorzugsweise an einer Fermenterabdeckung (10) angebracht ist und damit die Freiraumhöhe ($H_3$) zwischen Sensor und Substratniveau mit einem Freiraum-Höhensignal (FM) erfasst, welches einem Steuergerät (SG) der Steuereinrichtung zugeführt wird, wo durch Subtraktion von der Gesamthöhe ($H_0$) die aktuelle Füllstandshöhe ($H_1$) mit

$$H_1 = H_0 - H_3$$

ermittelt wird,

dass über einen Höheneinsteller am Steuergerät (SG) ein Höhen-Einstellsignal (HE) entsprechend einem Höhensollwert ($H_2$) für das Tauchrührgerät dem Steuergerät (SG) zugeführt wird, welches mit einem Höhen-Stellsignal (HS) den Höhenstellmotor (14) so ansteuert, dass das Tauchmotorrührgerät (6) diese Sollwerthöhe ($H_2$) (gemessen bis zur obersten Rührflügelspitze) einnimmt, und

dass mit einer überlagerten Steuerung als Sicherheitssteuerung gegen ein Austauchen der Rührflügel im Steuergerät (SG) ein Vergleich zwischen den Signalen der aktuellen Füllstandshöhe ($H_1$) und der im Steuergerät gespeicherten Sollwerthöhe ($H_2$) durchgeführt wird, dergestalt

dass für den Fall, dass die Füllstandshöhe ($H_1$) größer als die Sollwerthöhe ($H_2$) ist kein Steuereingriff erfolgt, und

dass für den Fall, dass die aktuell erfasste Füllstandshöhe ($H_1$') kleiner als die Sollwerthöhe ($H_2$) ist, unabhängig vom eingestellten Höheneinstellsignal (HE) ein selbsttätiger Steuereingriff so erfolgt, dergestalt

dass das Tauchmotorrührgerät (6) soweit nach unten abgesenkt wird, bis die aktuelle Einstellhöhe ($H_2$') des Tauchmotorrührgeräts (6) um einen Höhendifferenzbetrag X kleiner als die aktuelle Füllstandshöhe ($H_1$') ist, sodass

$$H_1' = H_2' + X$$

und damit die Rührflügel (7) bei einem Rührvorgang vollständig eingetaucht sind.

5. Biogasanlagen-Fermenter nach Anspruch 4, **dadurch gekennzeichnet, dass** der Höhendifferenzbetrag X am Steuergerät (SG) vorgebbar ist.

6. Biogasanlagen-Fermenter nach Anspruch 4 oder Anspruch 5, **dadurch gekennzeichnet, dass** der überlagerte Steuereingriff für die Eintauchsicherung der Rührflügel (7) jeweils beim Überschreiten einer X-Wertdifferenz durchgeführt wird.

7. Biogasanlagen-Fermenter nach einem der Ansprüche 1 bis 6, **dadurch gekennzeichnet, dass** das Steuergerät (SG) mit seiner Steuerfunktion in eine Anlagensteuerung und/oder Anlagenregelung integriert ist.

8. Biogasanlagen-Fermenter nach einem der Ansprüche 1 bis 7, **dadurch gekennzeichnet, dass** das Tauchmotorrührgerät (6) an einem vertikalen Aggregateträger (8) und mittels einer Seilwinde (9) motorisch höhenverstellbar ist.

9. Biogasanlagen-Fermenter nach Anspruch 8, **dadurch gekennzeichnet, dass** der Aggregateträger (8) im oberen Bereich in einem domartigen Serviceschacht (11) aufgenommen ist, in den das Tauchmotorrührgerät (6) nach oben verlagerbar ist.

**Claims**

1. A biogas plant fermenter comprising a vertically motor-adjustable, submersible motor stirrer, wherein during operation of the biogas plant, the fermenter (1) is filled to a level ($H_1$) with substrate (3) to be fermented, the submersible motor-driven stirrer (6) with its agitator blades (7) being fully immersed in the substrate (3),

**characterized in**

**that** a filling level gauge (15) is provided to detect the substrate (3) filling level ($H_1$) and to generate a corresponding filling level measuring signal (FM) as an actual level signal,

**that** a control device (SG) is provided to which the filling level measuring signal (FM) is transmitted and which if a lower filling level ($H_1$') is detected, actuates the height-adjusting motor (14) for the submersible motor stirrer (6) so as to lower it far enough for the agitator blades (7) to continue to be totally immersed in the substrate.

2. The biogas plant fermenter according to claim 1, **characterized in that** the filling level gauge (15) comprises a mechanically operating sensor with a floater and/or a displacer, each having a position transmitter and/or a limit value switch.

3. The biogas plant fermenter according to claim 1 or claim 2, **characterized in that** the filling level gauge (15) comprises an optical and/or acoustic and/or radioactive sensor, in particular an ultrasonic echosounder impulse device or a non-contact gamma radiator with counter tube chain and/or a hydrostatic tube for a bubbler system.

4. The biogas plant fermenter according to any of claims 1 to 3, **characterized in that** a sensor of the filling level gauge (15) is attached above the substrate level, preferably at a fermenter cover (10), so as to detect the free space height ($H_3$) between the sensor and the substrate level by way of a free space height signal (FM) which is transmitted to a control unit (SG) of the control device where by subtraction from the total height ($H_0$) the current filling level ($H_1$)

$$H_1 = H_0 - H_3$$

is determined,

that via a height adjuster at the control unit (SG) a height adjusting signal (HE) is transmitted to the control unit (SG) corresponding to a desired level value ($H_2$) for the submersible stirrer to actuate the height-adjusting motor (14) by way of a height-adjusting signal (HS) such that the submersible motor stirrer (6) assumes such desired value level ($H_2$) (measured up to the topmost agitator blade tip), and

that with a superimposed control as a safety control against the agitator blades emerging, a comparison is performed in the control unit (SG) between the signals of the current filling level height ($H_1$) and the desired value level ($H_2$) stored in the control unit, such

that in case that the filling level ($H_1$) is higher than the desired value level ($H_2$), no further control procedure is carried out, and

that in case that the currently sensed filling level height ($H_1$') is lower than the desired value level ($H_2$), an automatic control procedure occurs independently of the preset height adjustment signal (HE), such that the submersible motor stirrer (6) is lowered down far enough until the current setting height ($H_2$') of the submersible motor stirrer (6) is lower than the current filling level height ($H_1$') by a height differential amount X such that

$$H_1' = H_2' + X$$

and thus the agitator blades (7) are totally immersed in a stirring operation.

5. The biogas plant fermenter according to claim 4, **characterized in that** the height differential amount X can be preset at the control unit (SG).

6. The biogas plant fermenter according to claim 4 or claim 5, **characterized in that** the superimposed control procedure for the immersion safeguard of the agitator blades (7) is carried out every time as an X-value difference is exceeded.

7. The biogas plant fermenter according to any of claims 1 to 6, **characterized in that** the control unit (SG) with its control function is incorporated in a plant control.

8. The biogas plant fermenter according to any of claims 1 to 7, **characterized in that** the submersible motor stirrer (6) is height-adjustable by means of a motor at a vertical assembly holder (8) and by means of a rope winch (9).

9. The biogas plant fermenter according to claim 8, **characterized in that** the assembly holder (8) holder is accommodated in the upper region in a dome-shaped servicing well (11) into which the submersible motor stirrer (6) can be displaced upwardly.

**Revendications**

1. Fermenteur pour installations de biogaz, comprenant un agitateur à moteur submersible réglable en hauteur de manière motorisée,

le fermenteur (1) étant, lorsque l'installation de biogaz est en marche, rempli d'un substrat (3) à mettre en fermentation jusqu'à un niveau de remplissage ($H_1$), et l'agitateur à moteur submersible (6) ainsi que ses ailes agitatrices (7) étant entièrement immergés dans le substrat (3),

**caractérisé en ce**

**qu'**un dispositif de mesure de niveau de remplissage (15) est prévu, au moyen duquel est mesuré le niveau de remplissage ($H_1$) du substrat (3) et est produit un signal de mesure de niveau de remplissage (FM) correspondant, en tant que signal de valeur réelle de niveau,

**qu'**un dispositif de commande (SG) est prévu, auquel est transféré le signal de mesure de niveau de remplissage (FM) et qui, dans le cas où est mesuré un niveau de remplissage ($H_1'$) plus bas, commande le moteur de réglage de hauteur (14) de l'agitateur à moteur submersible (6) de telle sorte que celui-ci soit abaissé et que les ailes agitatrices (7) continuent à être immergées entièrement dans le substrat.

2.  Fermenteur pour installations de biogaz selon la revendication 1, **caractérisé en ce que** le dispositif de mesure du niveau de remplissage (15) comprend un capteur à fonctionnement mécanique avec un flotteur et/ou un corps de refoulement munis respectivement d'un capteur de position et/ou d'un interrupteur à valeur limite.

3.  Fermenteur pour installations de biogaz selon la revendication 1 ou la revendication 2, **caractérisé en ce que** le dispositif de mesure du niveau de remplissage (15) comprend un capteur optique et/ou acoustique et/ou radioactif, notamment un appareil d'impulsion ultrasonore de type sondeur acoustique ou un émetteur gamma fonctionnant sans contact, à chaîne de tube compteur et/ou un tube hydrostatique pour une méthode de mesure pneumatique.

4.  Fermenteur pour installations de biogaz selon l'une quelconque des revendications 1 à 3, **caractérisé en ce qu'**un capteur du dispositif de mesure du niveau de remplissage (15) est agencé au-dessus du niveau de substrat, de préférence sur un couvercle de fermenteur (10) et mesure ainsi la hauteur d'espace libre ($H_3$) entre le capteur et le niveau de substrat à l'aide d'un signal de hauteur d'espace libre (FM) transféré à un appareil de commande (SG) du dispositif de commande, dans lequel est calculée en soustrayant du niveau total ($H_0$)

$$H_1 = H_0 - H_3$$

la hauteur actuelle de niveau de remplissage ($H_1$), **en ce qu'**est transféré à l'appareil de commande (SG), par le biais d'un appareil de réglage de hauteur équipant l'appareil de commande (SG), un signal de réglage de hauteur (HE) correspondant à une valeur de hauteur de consigne ($H_2$) pour l'agitateur submersible, ledit appareil de commande guidant le moteur de réglage de hauteur (14) au moyen d'un signal de réglage de hauteur (HS) de telle sorte que l'agitateur à moteur submersible (6) prend cette hauteur de consigne ($H_2$) (mesurée jusqu'à la pointe la plus élevée de l'aile agitatrice), et

**en ce qu'**est réalisée, à l'aide d'une commande prioritaire agissant en tant que commande de sécurité contre une émersion des ailes agitatrices (SG), une comparaison entre les signaux du niveau actuel de remplissage ($H_1$) et de la hauteur de consigne ($H_2$) enregistrée dans l'appareil de commande, de telle sorte

que dans le cas où le niveau de remplissage ($H_1$) soit supérieur à la hauteur de consigne ($H_2$), il n'y ait pas intervention du dispositif de commande, et que dans le cas où le niveau de remplissage mesuré actuellement ($H_1'$) soit inférieur à la hauteur de consigne ($H_2$), indépendamment du signal de réglage de hauteur (HE) paramétré, le dispositif de commande intervienne de manière automatique, de telle sorte

que l'agitateur à moteur submersible (6) soit abaissé vers le bas jusqu'à ce que la hauteur de réglage actuelle ($H_2'$) de l'agitateur à moteur submersible (6) soit inférieure d'une différence de niveau X au niveau de remplissage actuel ($H_1'$), de sorte que

$$H_1' = H_2' + X$$

et que les ailes agitatrices (7) sont entièrement immergées en phase d'agitation.

5.  Fermenteur d'installations de biogaz selon la revendication 4, **caractérisé en ce que** la différence de niveau X peut être prédéterminée sur l'appareil de commande (SG).

6.  Fermenteur d'installations de biogaz selon la revendication 4 ou la revendication 5, **caractérisé en ce que** la commande prioritaire pour la sécurité d'immersion des ailes agitatrices (7) intervient à chaque dépassement d'une différence de valeur X.

7.  Fermenteur d'installations de biogaz selon l'une quelconque des revendications 1 à 6, **caractérisé en ce que** l'appareil de commande (SG) est intégré, avec sa fonction de commande, à une commande d'installation et/ou régulation d'installation.

8.  Fermenteur d'installations de biogaz selon l'une quelconque des revendications 1 à 7, **caractérisé en ce que** l'agitateur à moteur submersible (6) est réglable en hauteur de manière motorisée sur un support d'équipement (8) vertical et au moyen d'un treuil.

**9.** Fermenteur d'installations de biogaz selon la revendication 8, **caractérisé en ce que** le support d'équipement (8) est monté en partie supérieure d'une trémie d'entretien (11) bombée, dans laquelle l'agitateur à moteur submersible (6) est déplaçable vers le haut.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 8605277 U
- US 5867972 A
- JP 63188384 B
- DE 19732198 C1